# EUROPEAN PATENT SPECIFICATION

(11) **EP 3 116 420 B1**
(45) Date of publication and mention of the grant of the patent: **09.05.2018**
(21) Application number: 15712004.9
(22) Date of filing: 11.03.2015
(51) Int. Cl.: A61B 17/56

(54) **APPARATUS FOR TREATMENT OF PATELLOFEMORAL CONDITIONS**
VORRICHTUNG ZUR BEHANDLUNG VON PATELLOFEMORALEN LEIDEN
APPAREIL DE TRAITEMENT DE TROUBLES PATELLO-FÉMORAUX

(30) Priority: 11.03.2014 US 201461951469 P; 11.03.2014 US 201461951470 P
(43) Date of publication of application: 18.01.2017
(73) Proprietor: Cotera, Inc., Menlo Park, CA 94025 (US)
(72) Inventor: SHENOY, Vivek, Menlo Park, CA 94025 (US); GIFFORD III, Hanson, S., Menlo Park, CA 94025 (US); DE GOUY, Marine, Menlo Park, CA 94205 (US)
(74) Representative: KIPA AB
(86) International application number: PCT/US2015/019938
(87) International publication number: WO 2015/138594

(56) References cited:
- EP-A1- 2 452 641
- US-A1- 2011 213 466
- US-A1- 2013 150 977
- US-A1- 2013 304 208

## Description

### FIELD OF THE INVENTION

The present invention generally relates to the field of orthopedic prostheses and procedures. In particular, the present invention is directed to apparatus for treatment of patellofemoral conditions.

### BACKGROUND

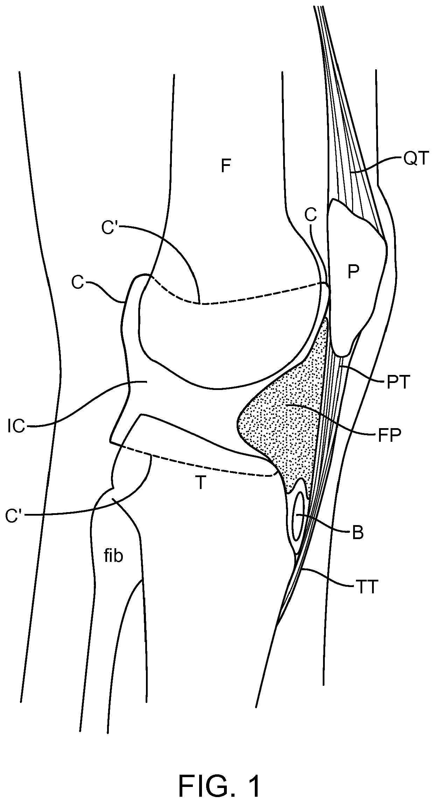

As the present invention is directed to apparatus for treatment of patellofemoral conditions, a basic discussion of the anatomy of the knee with a focus on the patellofemoral structures may assist in describing the various embodiments of the invention. FIG. 1 is a schematic portrayal in side view of a human knee. The bones of the knee joint comprise the femur (F), tibia (T), and patella (P). The fibula (fib) is another bone in the lower leg that attaches to the tibia. As primarily relevant to embodiments of the present invention, connective tissues of the knee joint include the patellar tendon (PT) and the quadriceps tendon (QT). The patellar tendon is also referred to as the patellar ligament as its primary function is to create a connection between bones, *i.e.,* the tibia and patella. The patellar tendon extends from the caudal (lower) extent of the patella to an attachment point on the tibial tuberosity (TT) of the tibia. The tibial tuberosity comprises a raised area on the anterior (forward) aspect of the tibia, caudally positioned (toward the foot or lower) with respect to the cranial (upper) end of the tibia. The quadriceps tendon extends from the cranial extent of the patella and joins with the quadriceps-femoris muscle.

The knee is a synovial joint, meaning that the bones are not directly joined but are surrounded by dense connective tissues forming an articular capsule (C) lined by a synovial membrane. The capsule defines a synovial cavity or intracapsular space (IC) that contains the articular cartilage of the joint (not shown) and synovial fluid that acts to reduce friction between the articular cartilages. The approximate extent of the capsule is indicated in FIG. 1 by dashed lines (C'). The quadriceps and patellar tendons lie outside the capsule. Also outside the capsule is the infrapatellar fat pad (FP). The fat pad is situated posteriorly and caudally with respect to the patella and joins with the patellar tendon on its posterior side over much of its length. The knee joint also includes a number of bursae to protect and facilitate movement between various bony and soft tissues. One of these bursae is the deep infrapatellar bursa (B), which allows for movement of the patellar tendon over the tibia. This bursa is positioned between the upper part of the tibia and the patellar tendon and lies in a pocket outside of the infrapatellar fat pad.

Because of the importance of the capsule in protecting and lubricating the articular cartilage, it is usually preferable, whenever possible in a knee intervention, to avoid penetrating the capsule. Because of the role of the infrapatellar fat pad in protecting the knee, it is also usually preferable to avoid dissecting the fat pad during knee interventions. Previously, removal of all or part of the fat pad was common in arthroscopic procedures in order to permit better visibility for the surgeon. However, it has been discovered that damage to the fat pad can lead to scarring, which can be painful and even crippling in some patients.

Treatments for various patellofemoral pathologies such as patellofemoral pain and patellofemoral osteoarthritis (PFOA) have been increasingly investigated. One early treatment, which involves anteriorization of the patellar tendon by a relatively invasive surgical procedure, was devised by Dr. Paul Maquet in the early 1960s. See, P. Maquet, 30 Revue Du Rhumatisme, No. 12, Dec. 1963, pp. 779-783, "Biomechanical Treatment of Patellofemoral Osteoarthritis, Advancement of the Patellar Tendon" (translated title). In this procedure, an iliac bone autograft is implanted under the patellar tendon to relieve pressure in the patellofemoral space. Later Dr. Maquet evolved his technique to cut the tibial tuberosity away from the tibia and reposition it. This became known as the Maquet Osteotomy, which has been performed on tens of thousands of patients over the years with positive results. See, e.g., Maquet, Biomechanics of the Knee, pp. 134-143 (pub. Springer-Verlag 1976). However, the Maquet Osteotomy is a highly invasive procedure, which carries with it all of the risks and costs associated with highly invasive orthopedic surgeries.

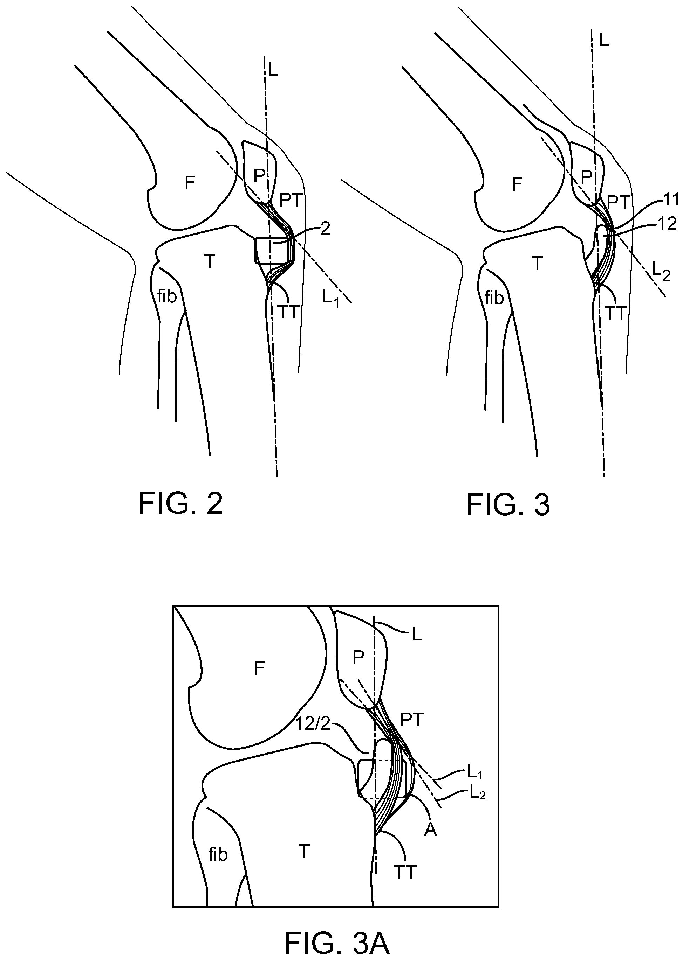

FIG. 2 is a schematic illustration of a relatively square bone implant 2 of the type proposed by Dr. Maquet implanted on the tibia (T) under the patellar tendon (PT). The patellar tendon is attached cranially to a caudal portion of the patella, and caudally to the tibia at the Tibial Tuberosity (TT). The natural line of action of the patella tendon would be generally along a line (L) extending between the two attachment points. Placing the bone implant 2 under the patellar tendon moves the patellar tendon anteriorly between its two attachment points and thus alters the line of action with respect to the patella. The new line of action (L₁), oriented more away from the patellofemoral space can reduce the pressure on that space. Said another way, anteriorizing the patellar tendon renders the angle between the patellar tendon and the quadriceps tendon more obtuse, reducing the resultant force pressing the patella against the femur.

However, the success of such a procedure may depend heavily on the configuration of the implant used. If the anterior, tissue-engaging surface of the implant is roughly perpendicular to the caudal face of the implant and/or parallel to the underlying surface of the tibia, it displaces the patellar tendon relatively directly anteriorly (perpendicular to the tibial surface) and creates an abrupt step at the caudal edge of the implant, which can produce a number of complications. First is the creation of an unsightly bump on the knee. This is not merely a cosmetic problem, as the bump may catch on clothing or other, harder objects that could cause bruising or injury in the course of daily activity. Second, such an implant could be extremely uncomfortable in certain common positions. For example, if a patient with such an implant were to kneel on that knee, all of the load would be placed on that implant, which could be painful and also damaging to the patellar tendon.

A third possible complication arises from the fact that an implant shaped such as implant 2 also pulls the patella caudally, creating an undesirable misalignment. This condition is referred to as "Patella Infera" or "Patella Baja". The symptoms of this misalignment can include pain on quadriceps contraction, inadequate quadriceps contraction, swelling, edema, joint stiffness, limited joint motion and limited patellar mobility.

Further, it may be desirable to maximize the area of the posterior surface of the implant that lies against the bone in order to spread the forces on the implant over as wide an area as possible. And, in order to minimize Patella Baja, it may be desirable to engage the patellar tendon as far cranially as possible without interfering with the patella or other tissues during knee movement. Yet, the space in which the implant is to be located, between the tibial tuberosity and the fat pad, bursa, and/or capsular tissues, is extremely limited. If the posterior surface of the implant extends too far cranially along the bone surface it may interfere with the fat pad, bursa, or joint capsule, causing pain or other complications. What is needed, therefore, are devices and methods for relieving patella-femoral pain due to osteoarthritis or other conditions that overcome the foregoing challenges.

In addition to the above, the present Applicant has disclosed implants for treating PFOA in United States Patent Publication US 2011/0213466, entitled "METHOD AND APPARATUS FOR FORCE REDISTRIBUTION IN ARTICULAR JOINTS," which defines the preamble of claim 1 and, more recently in United States Patent Publication US 2013/0211521, entitled "METHOD AND APPARATUS FOR ALTERING BIOMECHANICS OF ARTICULAR JOINTS." In certain embodiments therein disclosed, an implant portion is inserted underneath the patellar tendon, just cranial to the attachment of the patellar tendon to the tibial tuberosity in order to displace the patellar tendon anteriorly. The present invention is defined in claim 1, of which disclosed embodiments may reduce pressure of the patella against the femur, reducing patellar pain and patellofemoral cartilage wear. While such implants may also improve patellar tracking, or shift the location, angle or loading of the patella against the femur, there remains opportunities for further improvement of configurations heretofore disclosed to, for example, improve implant stability and effectiveness, facilitate placement and fixation.

### BRIEF DESCRIPTION OF THE DRAWINGS

For the purpose of illustrating the invention, the drawings show aspects of one or more embodiments of the invention. However, it should be understood that the present invention is not limited to the precise arrangements and instrumentalities shown in the drawings, wherein:
FIG. 1 is a schematic diagram illustrating anatomical features of a human knee joint in a side view;
FIG. 2 is a schematic illustration of an implant in accordance with early work of Dr. Paul Maquet;
FIG. 3 is a schematic illustration of an implant according to one disclosed embodiment;
FIG. 3A is a combined view with the embodiment shown in FIG. 3 superimposed over the implant shown in FIG. 2;
FIGS. 4A and 4B are diagrams illustrating profiles for tissue bearing surfaces in accordance with disclosed embodiments;
FIGS. 5A, 5B and 5C are a series of schematic illustrations showing disclosed embodiments implanted on the tibia at the knee, with the knee at flexion angles of about 0°, 45° and 90°, respectively;
FIGS. 6A, 6B and 6C show alternative examples, wherein FIG. 6A is a schematic illustration of the implant on the tibia, FIG. 6B is a perspective view of the implant, and FIG. 6C illustrates a further alternative, as would be seen along line C-C in FIG. 6B;
FIGS. 7A and 7B show an embodiment of the present invention, wherein FIG. 7A is a schematic illustration of the implant on the tibia, and FIG. 7B is a view through section A-A of FIG. 7A;
FIG. 7C illustrates an alternative view through section A-A of FIG. 7A;
FIG. 7D is a cross-sectional view through section D-D of FIG. 7B;
FIG. 8 is a schematic diagram of a human knee in side view illustrating positioning of an implant according to another disclosed embodiment;
FIGS. 9A, 9C and 9D are perspective views of further alternative examples employing supplemental fixation/support means;
FIG. 9B is a cross section through the displacement portion and part of the spanning section of the example of FIG. 9A;
FIG. 9E is a schematic illustration of an example such as shown in FIG. 9C in place on a portion of the tibia;
FIGS. 10A, 10B, 10C, 10D and 10E are various views of an alternative embodiment employing a separate fixation base member;
FIG. 10F is a schematic illustration of a further alternative embodiment, also employing a separate fixation base in place on a portion of the tibia;
FIGS. 11A, 11B, 11C, 11D and 11E are views of other alternative embodiments having differently shaped fixation portions;
FIGS. 12A and 12B are schematic illustrations of further alternative embodiments adapted for femoral fixation;
FIGS. 13A and 13B are views of an example of disclosed instrumentation useful in procedures for placement of implants disclosed herein;
FIG. 14 is a drawing of another embodiment viewed from the anterior aspect of an implant with a configuration generally as described herein;
FIG. 15 is a drawing of another embodiment viewed from the anterior aspect of an alternative implant including features similar to the embodiment shown in FIGS. 7A and 7B;
FIG. 16 is a drawing of another embodiment viewed from a cranial aspect of another alternative implant with a displacement portio having an increasing thickness;
FIG. 17 is a drawing of another embodiment viewed from an anterior aspect of a further alternative implant including features similar to the embodiments shown in FIGS. 9B and 9C; and
FIG. 18 is a drawing of another embodiment viewed from a caudal aspect of yet another alternative implant with features to facilitate positioning prior to fixation.

### DETAILED DESCRIPTION

Embodiments of the present invention employ an improved implant geometry with an appropriately curved cross-section to address drawbacks of some prior devices and procedures, such as an unsightly and uncomfortable bump, concerns about tissue damage, and the caudal movement of the patella as previously discussed. Other embodiments of the present invention employ supplemental support/fixation means and specially shaped fixation portions to facilitate implantation, increase fixation security and resist torqueing forces. Embodiments of the present invention may be used in less invasive methods for treatment of patellofemoral conditions.

FIG. 3 schematically illustrates features of the embodiments in this disclosure. The schematic side view of FIG. 3 helps to illustrate the positioning and curved shape of the bearing surface 11 and displacement portion 12 of embodiments disclosed herein. The bearing surface 11 is the surface of the displacement portion 12 in contact with the patellar tendon. Line L₂ shows the approximate line of action of the patellar tendon after repositioning over displacement portion 12. To simplify FIG. 3 for discussion purposes, fixation means and other implant structures such as the fixation portion and spanning section discussed in more detail below are not called out. The various fixation and support structures discussed below facilitate the cantilevering of displacement portion to better accommodate soft tissue structures while properly positioning the bearing surface as discussed in more detail below.

In general, implants according to embodiments of the invention will be configured and dimensioned to displace the tissue targeted for treatment by between about 5 mm to about 30 mm from the natural, anatomical tissue path. In some embodiments, the displacement will be greater than about 10 mm. Overall displacement amounts can be set through a combination of shape and size of the fixation portion, spanning section and displacement portion of the implant as previously described. Working within those parameters, it has been discovered that by shaping the bearing surface at least approximately as a quarter-circle in cross-section with a minimum radius of about 8mm, caudal biasing of the patella can be reduced. Further flattening the curvature of the bearing surface, by increasing radius, or making the surface more oval, elliptical, hyperbolic, or of another complex shape, can further reduce caudal biasing, but space limitation arising from the anatomy and need for a minimum displacement to achieve therapeutic effects may limit the amount of such flattening that may be applied to the implant. In addition, the displacement portion and/or bearing surface may be shaped and dimensioned to provide different magnitudes of displacement at different points along the surface such that the tissue is displaced different amounts at different joint positions, e.g. at different points in the gait cycle.

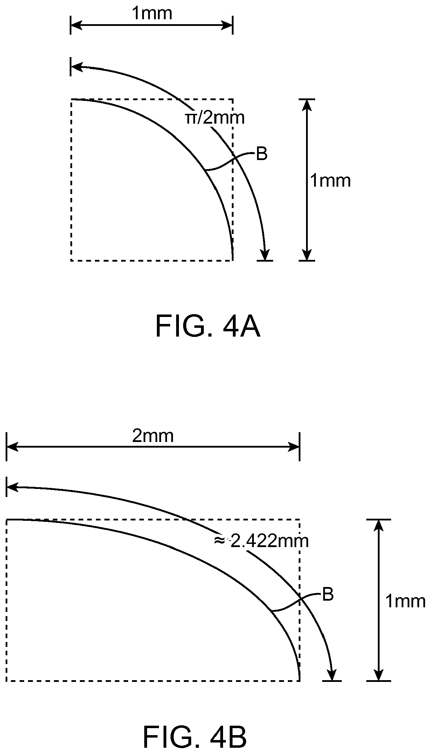

As shown in FIG. 4A, for a bearing surface (B) with a depth and length of 1mm in each dimension, the patella is pulled caudally by only about (Π/2 - 1) or 0.57 millimeters. In an alternative embodiment, the bearing surface curve may be flattened even further with a length of about 2mm while maintaining the 1mm depth, as shown in FIG. 4B. In this embodiment, the caudal displacement of the patella would be less than half of the anterior displacement. The generally elliptical shape of the bearing surface causes such an implant to extend generally twice as far cranially as it does anteriorly and thus would pull the patella caudally by an amount approximately equal to 0.42 times the anterior displacement. Caudal displacement with an elliptically shaped bearing surface may be estimated based on a corresponding elliptical circumference. For example, using an "ellipse calculator" (*e.g*. as available online at http://www.cleavebooks.co.uk/ scol/callipse.htm) and selecting a major axis of 4 and a minor axis of 2, a circumference of 9.69 can be determined. Dividing the circumference by 4 gives 2.422 (approximately one quarter of the elliptical circumference corresponding to the overall length of the bearing surface). With this information, it may be estimated that a curved bearing surface with a cranial-caudal length of 2 cm and an anteriorization of 1 cm will pull the patellar tendon caudally approximately 0.42cm.

A geometry as described in the preceding paragraphs should dramatically reduce the complications caused by patella baja from square or steeply profiled implants. For example, a prior art implant with a square cross-section, such as shown in FIG. 2, would pull the patella caudally by approximately one millimeter for each millimeter of anteriorization; about twice the amount of caudal displacement created by embodiments disclosed herein for the same amount of anteriorization.

In certain embodiments, the bearing surface 11 will be positioned with its outer most point (apogee) at a perpendicular distance from the surface of the tibia below it of about 0.3-3 cm, or more typically about 0.5-1.5 cm for an implant configured to treat an average adult knee. The width of the bearing surface in the generally cranial-caudal direction will be about 0.5-3.0 cm, or more typically about 1.0-2.5 cm. While distance from the tibia to the apogee of the bearing surface can equal the bearing surface width, in some embodiments the width will be greater than that distance, about 1.1-3.0 times greater, or more typically about 1.5-2.0 times greater. Further alternative embodiments may employ bearing surfaces with compound curvatures comprising elements of FIGS. 4A and 4B as previously discussed.

A further physiologic benefit to an implant with the curved geometry as described is that the forces pressing the patella against the femur are highest when the knee is bent, such as when a person is climbing stairs. As shown in FIG. 3A, where an embodiment of the present invention is superimposed over a prior implant, the shape of the present invention is more effective in flattening the angle between the patellar tendon and the quadriceps tendon when the knee is bent. It also reduces the focal stress on the sharply angled portion of the patellar tendon caused by such prior implants, especially when the leg is straight. The extreme caudal positioning of the prior implant 2 is indicated at (A) in FIG. 3A.

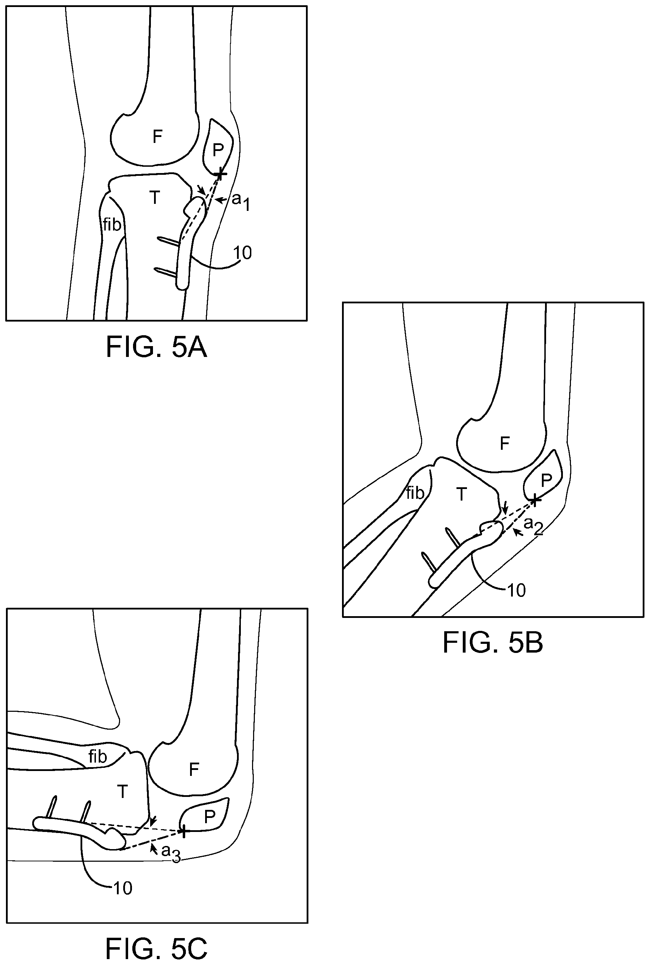

The beneficial effect of embodiments of the present invention as related to knee flexion are further illustrated in FIGS. 5A-C. From these figures, it can be seen that the angle α of anteriorization is increased from the natural line of the patella tendon from α₁ with the knee fully extended, to α₂ at partial flexion, up to α₃ at 90° flexion in FIG. 5C, where α_{1<} α_{2<} α₃. The unloading provided by the implant thus increases with knee flexion, providing the greatest relief when the patella is maximally loaded.

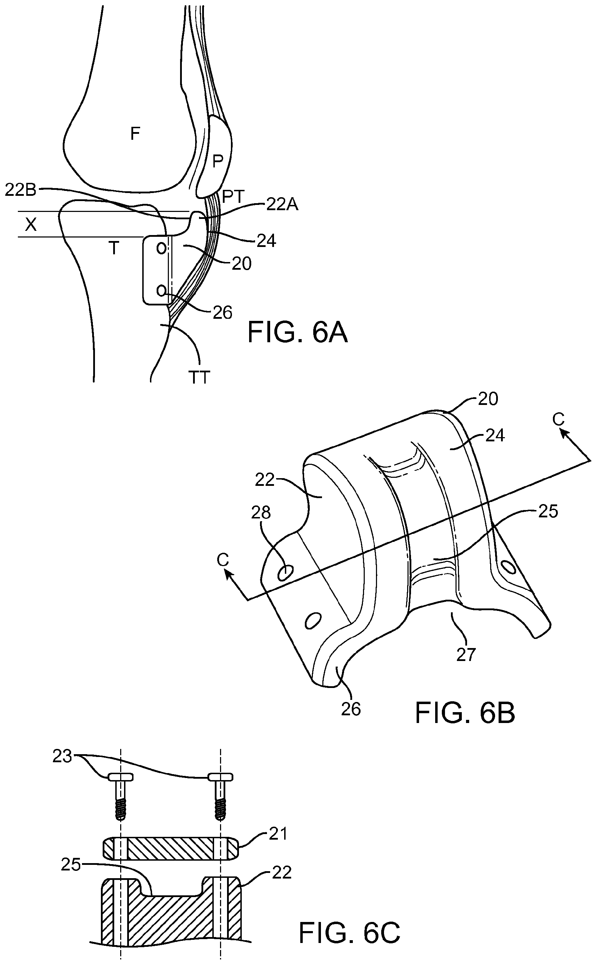

Another alternative embodiment of the present invention is shown in FIGS. 6A and 6B. While typically it would be preferable to position the fixation portion more caudally to locate the fixation means such as screws more distant from the joint capsule and other sensitive structures of the joint, in some patients and in some clinical situations this may not be possible, or fixation adjacent to the displacement portion may have other advantages. In this embodiment, implant 20 may have a displacement portion 22 with a bearing surface 24. The bearing surface may be curved as described above in connection with FIGS. 4A and 4B. The bearing surface also may be optionally provided with a concave groove or channel 25 extending in the cranial-caudal direction to assist in guiding the displaced tissue as it passes thereover. However, the same embodiment also may be provided without the channel. Implant 20 also may have a fixation portion 26 with a bottom surface having a slight concavity 27 in the cranial-caudal direction configured to be seated on the tibia just cranially of the tibial tuberosity. Fixation means 28 such as screw holes, spikes or bone ingrown facilitating elements may be included in fixation portion 26. Persons of ordinary skill in the art will appreciate that the features of the concave groove or channel 25 and/or bottom surface concavity 27 may be employed with other embodiments as described herein and are not restricted to use with implant 20.

As shown in FIG. 6A, and as employed by embodiments of the invention disclosed herein, displacement portion 22 includes a cantilevered portion 22A extending in the cranial direction, forming an undercut region 22B on the inferior (bone facing) side of cantilevered portion 22A, cranially of fixation portion 26. Cantilevered region 22A is configured to extend cranially over the anterior surface of the tibia and the overlying fat pad such that the cranial edge of bearing surface 24 extends a distance X of about 5-30 mm, more preferably about 10-25 mm, and most preferably about 15-22 mm from the cranial edge of fixation portion 26. This facilitates engagement of bearing surface 24 with the patellar tendon as far in the cranial direction as possible without interfering with the patella or femur, while undercut region 22B provides a space in which the fat pad, capsular ligaments or other soft tissues may reside. By engaging the patellar tendon further in the cranial direction, the displacement force applied to the patella has less of a caudal component, reducing "Patella Baja".

In another alternative, a cover member 21 may be provided as shown in FIG. 6C to protect and retain the tendon when it is received in channel 25. Cover 21 is securable over bearing surface 24, such as with screws 23.

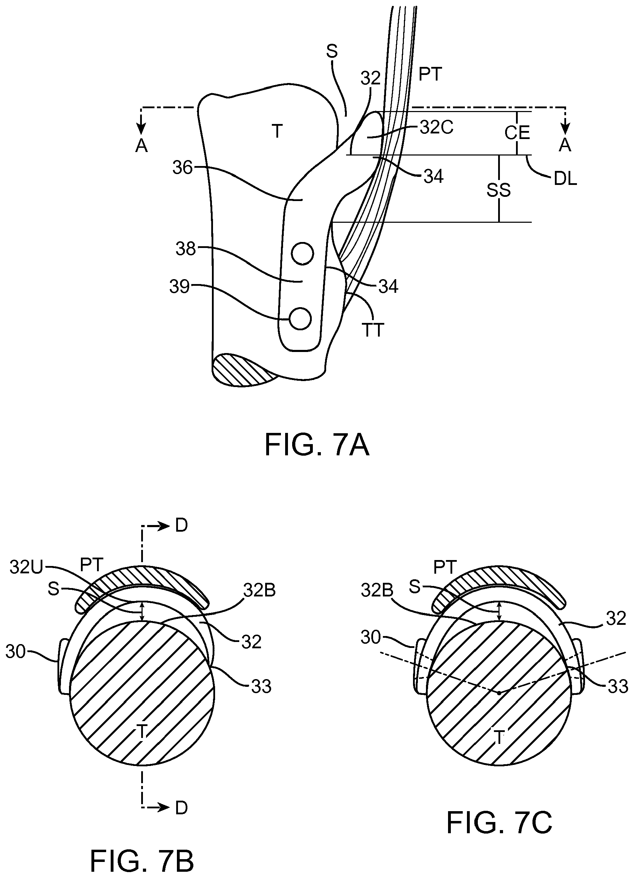

An embodiment of the present invention is shown in FIGS. 7A and 7B. In this embodiment, implant 30 may have a displacement portion 32 with a bearing surface 34. Bearing surface 34 also may be curved as described above in connection with FIGS. 4A and 4B. Displacement portion may be supported and positioned by spanning section 36, which is in turn supported by fixation portion 38. Spanning section 36 and displacement portion 32 may be configured and dimensioned to provide varying amounts of cantilever for the bearing surface 34. Such a cantilever can provide clearance for the fat pad and/or other critical tissues behind the implant. Fixation means 39 such as screw holes, spikes or bone ingrown facilitating elements may be included in fixation portion 38.

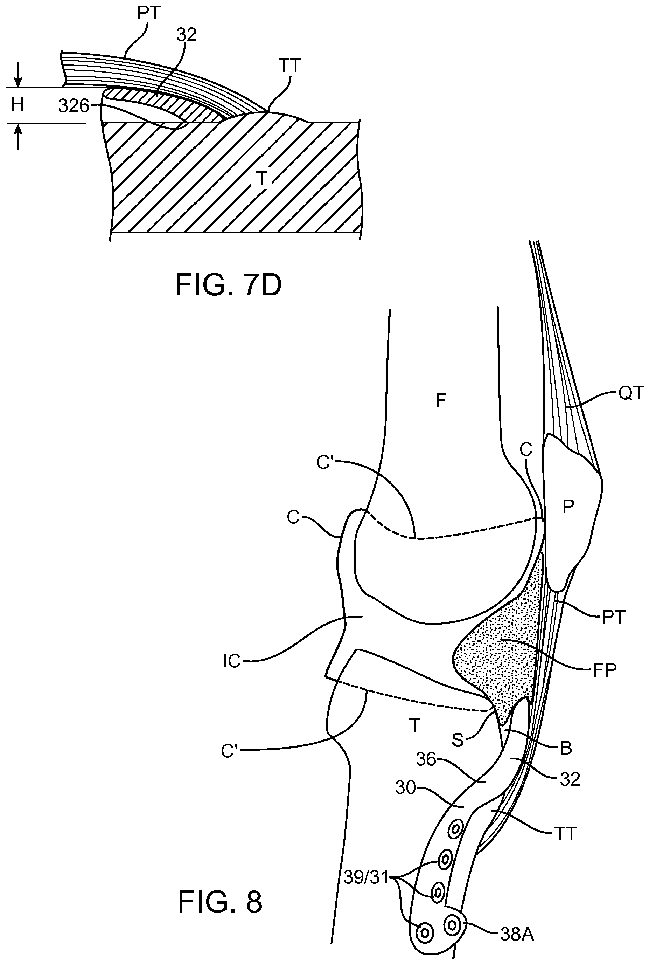

Referring to FIGS. 7B and 7D, it can be seen that displacement portion 32 can be provided with a concavity on the bone-facing posterior side of displacement portion 32 that spaces the displacement portion away from the tibial, forming a space (S) therebetween with a height indicated by the double arrow. Space S preferably has a maximum height between an underside 32U of displacement portion 32 and the surface of the tibia in a range of about 5-25 mm, or more typically about 10-20 mm, in order to accommodate the fat pad and other tissues beneath the displacement portion 32. Displacement portion 32 has a supporting section 32B, as described above, that sits in engagement with the tibia in the space between the tibial tuberosity TT and the caudal edge of the fat pad, thereby supporting displacement portion 32. Height (H), the perpendicular distance between the bone engaging surface of supporting section 32B and the apogee of bearing surface 34, as also described above, is shown in FIG. 7D.

To fit displacement portion 32 within the available space, with reference again to FIG. 7A, supporting section 32B preferably extends in the cranial-caudal direction a distance (SS) of no more than about 20 mm, usually being about 5-15 mm, more preferably about 8-12 mm. The cranial end 32C of displacement portion 32 preferably extends a distance (CE) of about 5-30 mm, typically about 10-25 mm, or more typically about 15-22 mm in the cranial direction from the upper (cranial) extent of supporting section 32B, which lies against the tibia approximately at dimension line (DL) in FIG. 7A The concave shape of the posterior side of displacement portion 32 causes its lateral margin 33 to extend around the lateral side of the tibia (if the fixation portion of the implant is mounted on the medial side). In some embodiments, for example as shown in FIG. 7B, lateral margin 33 may be configured to contact the tibia to provide additional support for the displacement portion and bearing surface in resisting forces applied by the patellar tendon, particularly at high flexion angles. An additional fixation portion on the lateral end of the implant is shown in FIG. 7C, which provides additional support and stabilization for the implant, but which does not form part of the present invention.

FIG. 8 illustrates an embodiment of the present invention, such as implant 30 described above, after implantation on the tibia. In this embodiment, implant 30 also employs an extended fixation portion 38A for enhanced torque resistance as described in more detail below. Fixation portion 38A may have an extension portion that wraps around either the anterior or posterior side of the tibia, or both, to further stabilize the implant. Multiple screw holes 39 and bone screws 31 are used as dictated by patient anatomy and clinical factors such as condition of the bone.

Placement and fixation of an implant according to embodiments of the present invention can often be accomplished through a single surgical incision adjacent the patient's knee. The implant is then placed through the incision with the displacement portion inserted under the patellar tendon cranially with respect to its attachment point to the tibia at the tibial tuberosity. A therapeutic location that is a target area for placement of the displacement portion includes the caudal pocket below the infrapatellar fat pad containing the infrapatellar bursa (B). Reference letter (B) is provided in FIG. 8 to identify the target area, but the bursa itself is not shown because in some situations it may be necessary to remove part or all of the bursa to accommodate the implant. However, unlike the articular capsule or the infrapatellar fat pad, there are not significant potential negative indications associated with removal or dissection of the infrapatellar bursa.

Placement of the implant as shown for example in FIG. 8, allows the implant to be placed and fixed through a single incision without penetrating the capsule (C) or dissecting the infrapatellar fat pad (FP) or separating it from its attachment along the posterior of the patellar tendon (PT). Of course, depending on the shape and size of the implant as clinically determined by the surgeon, it may be necessary to push on and somewhat reposition the fat pad as indicated in FIG. 8 as compared to FIG. 1. Also, as previously described, the smooth, curved shape of the bearing surface 34 and displacement portion 32 moves the patella anteriorly and away from the femur while limiting the amount of movement caudally, thus reducing or avoiding a Baja effect. The shape of implant 30 also effectively avoids and preserves the natural attachment point of the patellar tendon (PT) to the tibia at the tibial tuberosity (TT).

FIG. 8 also further illustrates how the shape of the spanning section 36 and displacement portion 32 provides a cantilevered bearing surface 34 to define space (S) under the implant to accommodate the fat pad and its attachment to the side of the tibia. The cantilevered portion of the implant may be configured to deflect under high loading conditions to reduce strain on the tendon. Such deflection may be engineered into the implant by selection of shape, thickness and material so as to allow the implant to flex, or more active means such as springs or hydraulic cylinders may be used. In further alternatives, bearing surfaces of implants according to embodiments of the invention may include resilient elements such as fluid filled pillows and/or pressure control volumes utilizing check or relief valve systems.

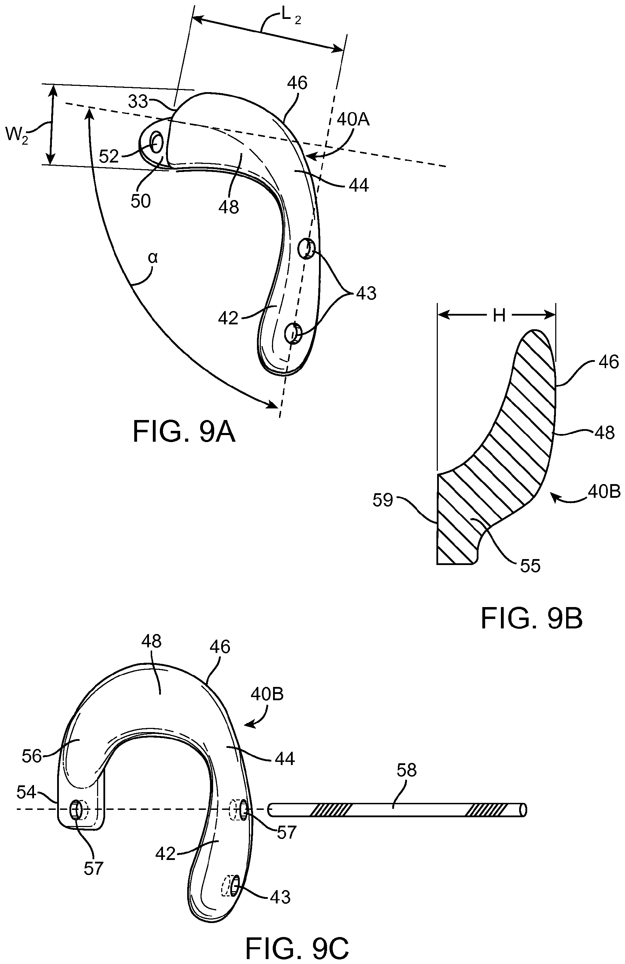

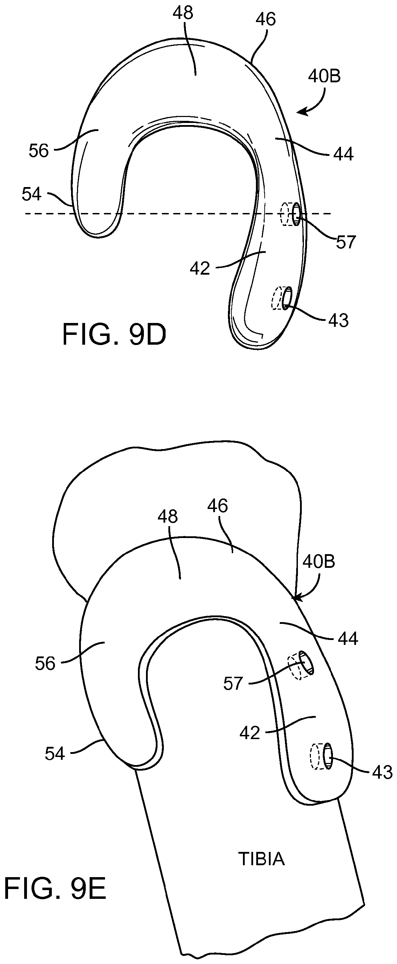

FIGS. 9A and 9C illustrate further examples employing supplemental support and fixation elements 50 and 54. Implants 40A and 40B each include fixation portion 42 with fixation means such as bone screw holes 43, spanning section 44 and displacement portion 46 with bearing surface 48. With respect to implant 40A, fixation portion 42 is generally straight and elongated, being configured for positioning in general alignment with the tibial shaft on the medial or anterior-medial side of the tibia. A first bone engaging surface is provided on the bone facing side of the fixation portion 42. Holes 43 are preferably positioned in approximate alignment with a longitudinal centerline of fixation portion 42.

Displacement portion 46, is configured and dimensioned to be positioned under the patellar tendon caudally separated from the insertion point of the tendon in the tibia. The displacement portion 46 is configured to atraumatically engage the tendon and displace it anteriorly relative to the tibia. The displacement portion 46 has a length in the lateral-medial direction generally selected to accommodate the full width of the tendon so that the tendon remains engaged along its entire width as it slides on the displacement portion. Displacement portion 46 preferably has a convex curvature on its outer tissue-engaging surface (bearing surface 48), preferably being curved at least around an axis generally parallel to the tibial shaft, usually being curved also around an axis perpendicular to the tibial shaft, and more preferably being spherical or partially spherical. Alternatively the bearing surface may have other curvatures such as elliptical, parabolic, logarithmic spiral, or other complex curvature. Displacement portion 46 has a width in the caudal-cranial direction selected so that it does not interfere with the patella or engage the insertion point of the tendon, typically being less than its length.

Spanning section 44 interconnects fixation portion 42 and displacement portion 46. Spanning section 44, in the embodiment illustrated, extends cranially and laterally from fixation portion 42 to displacement portion 46, forming a curve of about 90° about a dorsal-ventral axis. Where fixation portion 42 is configured for attachment to a more medial aspect of the tibia, spanning section 44 will extend ventrally as well as cranially and laterally from fixation portion 42, preferably being curved about an axis generally parallel to the tibial shaft. Displacement portion 46 appropriately displaces the patellar tendon in cooperation with the fixation portion 42 and spanning section 44. An angle α between the displacement portion 46 and the fixation portion 42 (as measured at the intersection of the center line axes of the two portions in the top view of the implant in FIG. 9B) may range from about 80 degrees to 135 degrees, more specifically from about 85 degrees to 120 degrees, and in some examples about 90 degrees to 110 degrees.

The width W₂ (generally cranial-caudal direction) of the displacement portion 46 is generally selected to provide a broad area of contact with the tendon to spread the force and reduce wear, while not interfering with the patella or the tendon insertion point throughout the full range of joint motion. Width W₂ may thus range from about 10 mm to 25 mm, more specifically about 12 mm to 20 mm, and in some embodiments about 14 mm to 18 mm. The length L₂ (generally medial-lateral direction) of the displacement portion 46 is selected so that the displacement portion extends under the full width of the tendon so that the entire width of the tendon remains in engagement and displaced the desired amount throughout the range of joint motion. Length L₂ may thus range from about 20 mm to 50 mm, more specifically about 25 mm to 45 mm, and in certain embodiments about 30 mm to 40 mm.

Positioned at the end of displacement portion 46 on implant 40A is supplemental support and fixation tab member 50 with at least one bone screw hole 52. In some embodiments, implant 40A may be generally shaped in a manner similar to implant 30 of FIG. 7B, with tab member 50 disposed at the lateral margin 33 of the implant having a second bone engaging surface in contact with the tibia. In other embodiments, the shape may be generally reversed such that tab member 50 would be disposed at a medial margin of the implant displacement portion. In a further embodiment shown in FIG. 9D, tab member 50 has no bone screw hole, but simply provides additional surface area resting against the tibial surface to stabilize the device and to more widely distribute the pressure of the device due to the force of the patellar tendon against the device.

As illustrated in FIG. 9B, implant 40B also includes a supporting section 55 extending along a caudal extent of displacement portion 46, into spanning section 42 and merging into the first bone engaging surface 59 of fixation portion 42.

Supporting section 55 rests on the surface of the tibia between the tendon insertion point and the fat pad and/or capsular tissue. The cranial-caudal length of first bone engaging surface 59, i.e., the approximate distance the cranial extent of the supporting section, to the caudal end of fixation portion 42, is preferably greater than the distance from the cranial extent of the supporting section to the cranial edge of displacement portion 46. The appropriate distance ratios between these two regions increases the moment arm resisting torqueing force applied by the patellar tendon through the cantilevered displacement portion 46 to help fix the implant in place and resist loosening over time due to the cyclic torqueing forces applied by knee flexion and extension.

Displacement portion Height (H), shown in FIG. 9B, is the perpendicular distance from the apogee of bearing surface 48 to the first bone engaging surface 59. Height (H) directly effects the amount of displacement of the tendon achievable with the implant. In general terms, the displacement distance will approximately equal Height (H) minus the normal anatomical distance between the patellar tendon and the tibial surface below it at the location of the displacement portion when implanted.

The position of tab member 50 with respect to fixation portion 42 may necessitate a second surgical incision site when placing implant 40A. In order to provide supplemental fixation and support means without necessitating a second incision site, means such as shown in FIG. 9C for implant 40B may be alternatively employed. In this embodiment, displacement portion extension 56 extends the displacement portion in a caudal direction around the lateral side of the tibia (if the fixation portion 42 is mounted to the medial side of the tibia). Supplemental support and fixation tab 54 is disposed at the caudal and/or lateral margin of the extended displacement portion and provided with at least one fixation hole 57. To allow for placement and fixation from a single incision site, fixation hole 57 is configured to accommodate fixation rod 58 and is aligned with a corresponding fixation hole 57 in fixation portion 42. Fixation rod 58 may comprise a threaded rod or elongated bone screw, and fixation hole 57 may be threaded so as to receive the threaded tip of the fixation rod. In an alternative embodiment, fixation rod 58 may be threaded over its entire length with a pointed distal end and a bone screw head at the proximal end adapted to receive a torqueing tool such as a hex driver.

Placement of implant 40B is achieved by positioning the fixation portion on one side of the tibial tuberosity with the extended displacement portion 56 extending around the attachment of the patellar tendon to the tibia and back down caudally on the opposite side of the tibial tuberosity. With fixation holes 57 thus aligned on opposite sides of the tibia, fixation rod 58 may be inserted through the same surgical incision and through a portion of the tibia to fix both holes 57 in a single operation. Additional fixation screws may be placed in other holes 43, again through the same surgical incision. A specialized drill guide might be employed to ensure accurate alignment while drilling the hole.

A supporting section as generally described above may be incorporated into other embodiments disclosed herein to facilitate locating the fixation portion (and in particular bone screw site) at a distance from the area where displacement portion acts to allow for easier placement of the device, without a need to place fixation elements such as nails or screws under or close to the patellar tendon, the joint capsule or the infrapatellar fat pad. In this manner, cantilevering of at least a part of the displacement portion, with the bearing member, over the tibia is facilitated to allow for a portion of the fat pad and its attachment to the tibia to be received thereunder. It also means that the displacement element can be appropriately rounded and smooth, without any surface roughness or disturbances due to fixation elements. And although the fixation portion is at a distance from the displacement portion, much of the force from the patellar tendon is transmitted through the supporting section directly onto the tibia behind it. Further, by extending the fixation portion caudally down the tibia relative to the supporting section the leverage applied by the fixation screws is increased so as to counter any tendency of the displacement portion to be tilted toward the tibia under the forces exerted by the patellar tendon.

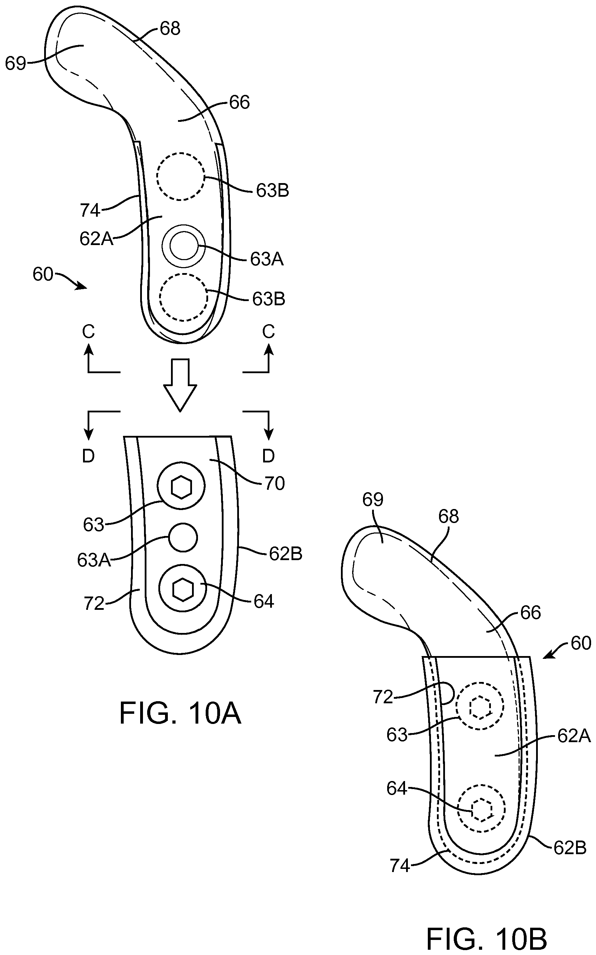

Depending on patient anatomy and other clinically determined parameters, placement of an implant according to embodiments of the present invention may present a challenge because of the torqueing forces exerted on the displacement portion after insertion under the patellar tendon, even before fixation means, such as bone screws, are secured. Such torqueing forces would tend to lift the fixation portion away from the bone surface to which it was to be affixed. In this situation, a separate fixation base may be employed as shown, for example, in FIGS. 10A-E. In this alternative embodiment, implant 60 has a fixation portion that comprises a body member fixation portion 62A and a base member fixation portion 62B. FIG. 10A shows the part unassembled before placement of the body member fixation portion 62A, and FIG. 10B shows the assembled parts as they may appear after placement.

As shown in FIGS. 10A and 10B, implant 60 also includes spanning section 66 and displacement portion 68 with bearing surface 69 generally as previously described. In one embodiment, screw holes 63, configured to receive bone screws 64, are provided only in base member fixation portion 62B. In a further alternative embodiment, illustrated only in FIG. 10A, additional fixation screw holes 63A may be provided in both the base member and body member fixation portions and positioned so that the holes align when the body member is received in the base member. In another alternative embodiment, bone screw access holes 63B may be provided as discussed further below. Access holes 63B are shown in FIG. 10A in dashed lines as optional features.

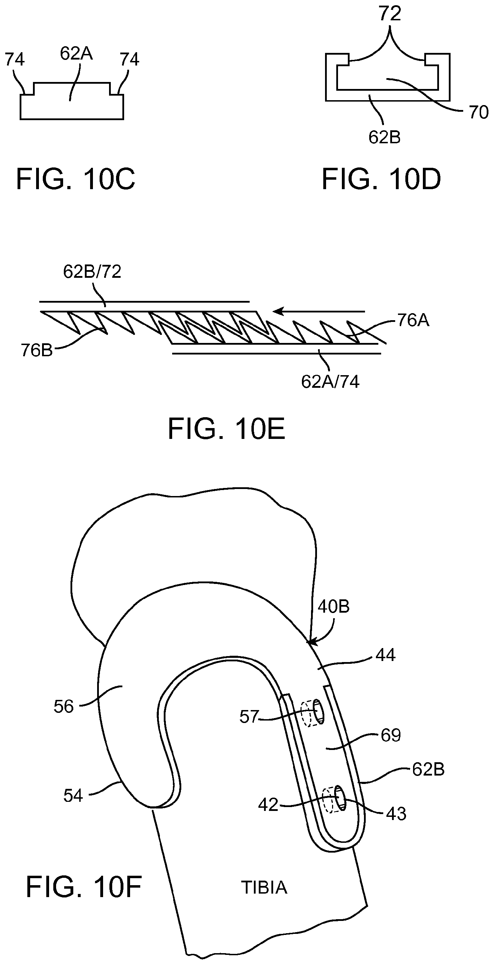

Body member fixation portion 62A and base member fixation portion 62B are provided with complementary, mating shapes to permit them to be securely fitted together. Persons of ordinary skill in the art may select from various complementary shapes, one example of which is shown in FIGS. 10C and 10D, which are end views at lines C-C and D-D, respectively, in FIG. 10A. In this exemplary embodiment, base member fixation portion 62B defines a channel 70 with retaining edge 72 that extends therearound. The complementary shape of body member fixation portion 62A is provided by guide channel 74, which in this exemplary embodiment extends around the caudal end and onto both sides of the body member fixation portion 62A. In other embodiments, separate mating features may be provided only on the sides, not extending around the caudal end of the implant.

The two-piece design of an embodiment such as implant 60 permits the base member fixation portion 62B to be first secured at a selected location without an eccentric or torqueing forces applied by the target tissue through the displacement portion 68. Fixation means such as holes 63 and bone screws 64 may be used to secure the base member fixation portion 62B. Once proper placement is confirmed, displacement portion 68 may be inserted under the target tissue, such as the patellar tendon, and then base member fixation portion 62A inserted into base member fixation portion 62B with a relatively straightforward sliding action as indicated by the arrow in FIG. 10A to provide a combined implant generally as shown in FIG. 10B. Screw holes 63 and screws 64 are shown in phantom lines in FIG. 10B because they are covered by body member fixation portion 62A. For this reason screws 64 preferably are low profile screws with flat heads to avoid interference with the body member when inserted into the base member. Channel 74 is also shown in phantom lines because it is received behind edge 72.

Various locking means for securing the body member to the base member are possible. One such locking means embodiment is schematically illustrated in FIG. 10E. In this illustrative embodiment, inter-engaging teeth 76A and 76B are provided on the facing surfaces, respectively, of the body member channel 74 and the base member retaining edge 72. When body member is inserted into the base member as indicated by the arrow in FIG. 10E, the inter-engaging teeth act in a ratchet-like manner, permitting insertion but preventing removal. In some embodiments, the teeth may themselves be formed with resiliency to permit insertion. In other embodiments, the teeth may be relatively short with less resiliency to permit insertion provided by elastic deformation of base member fixation portion 62B and retaining edge 72. For even greater fixation security, after the body member is fully received in the base member, additional bone screws may be inserted through optional, additional fixation screw holes 63A, which become aligned as described above.

It will also be appreciated by persons of skill in the art, that inter-engaging teeth or other ratchet-type locking means may be difficult to disengage if it becomes necessary to remove or reposition the base member during the initial implant procedure or a later intervention. Disengagement may be achieved, for example, by deformation of base member fixation portion 62B and retaining edge 72. In one alternative, bone screw access holes 63B may be provided in body member fixation portion 62A as shown in FIG. 10A. Access holes 63B are positioned to align with bone screw holes 63 in base member fixation portion 62B when the body member is received in the base member after implantation. Using access holes 63B, bone screws 64 may be removed without separating the body member from the base member.

In another alternative embodiment, inter-engaging or ratchet-type locking means is not provided. Instead, locking means may be provided by one or more additional fixation screw holes 63A. In such an embodiment, body member fixation portion 62A may be freely inserted and removed from base member fixation portion 62B once the base member is installed. The complementary shape of the mating parts as described initially carries the torqueing force of the target tissue acting on displacement portion 68 and then the two members are locked together using bone screws through one or more additional fixation screw holes 63A. Bone screw access holes 63B also may be included as desired to provide further removal options.

FIG. 10F shows an additional alternative body member-base member geometry, with a curved interface between the parts which allows adjustment for any variation in the angle of the tibial surface against the fixation portion. This allows the body member 69 to be positioned so that the posterior edge of the displacement section rests firmly against the tibia as the fixation screws are tightened.

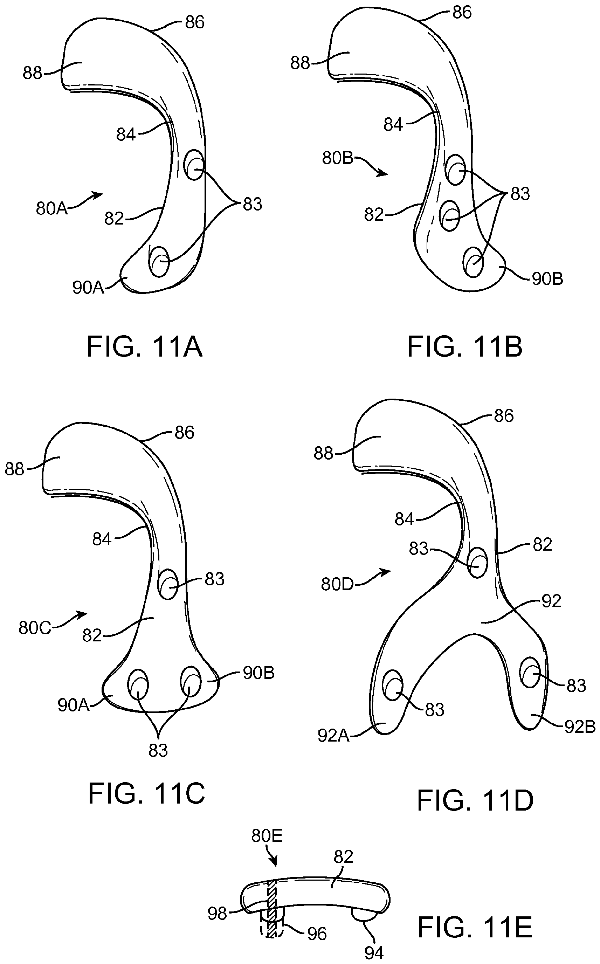

As mentioned above, torqueing and other complex forces applied to the fixation portion through the target tissue acting on the displacement portion may be significant. In order to better resist such forces various supplemental fixation and support embodiments may be provided. Two such exemplary embodiments have been described above in connection with FIGS. 9A and 9C. Additional exemplary embodiments are shown in FIGS. 11A-E. Implants 80A-E of FIGS. 11A-E each include fixation portion 82 with screw holes 83, spanning section 84 and displacement portion 86 with bearing surface 88 generally as previously described. In addition to these basic structures, implant 80A may include extension portions extending from fixation portion 82 in a direction generally transverse to that of fixation portion 82. For example, as shown in FIG. 11A, an extension portion 90A may be configured to extend generally in the same direction as the displacement portion, *e.g.,* if the fixation portion 82 is mounted on the lateral side of the tibia, in a medial direction (or angled medially and caudally), across the anterior surface of the tibia transverse to the longitudinal axis of fixation portion 82, but caudally of the tibial tuberosity. In another alternative, as shown in FIG. 11B, implant 80B includes extended fixation portion 90B, which extends in a direction opposite from the displacement portion 86, *e.g.* laterally, or laterally and caudally, relative to fixation portion 82 so as to extend around the lateral side of the tibia. In another example, if the fixation portion 82 is mounted to the medial side, extended fixation portion 90B may be configured to extend posteriorly further around the medial and/or posterior side of the tibia. Extended fixation portions 90A and 90B create a wider base for fixation portion 82 and thus provide greater resistance to torqueing forces as described. Extended fixation portions 90A and 90B generally will be configured and dimensioned to match the shape of the tibia in the area of contact.

An extended fixation portion configured and positioned as extended fixation portion 90A will help to resist torqueing applied to the displacement portion 86 by creating a greater surface bearing against the bone at a greater distance from the center of rotation of the torqueing force, which will lie approximately along a centerline of fixation portion 82. An extended fixation portion configured and positioned as extended fixation portion 90B will help to resist torqueing force applied to the displacement portion 86 by creating a greater lever arm through which a bone screw in fixation holes 83 may act to resist the torqueing force. In some situations it may be desirable to utilize both extended fixation portions 90A and 90B to achieve the benefits of both approaches. FIG. 11C shows exemplary implant 80C employing both laterally- and medially-extended fixation portions 90A, 90B on one device.

Depending on patient anatomy, it may be desirable to provide a fixation portion that wraps farther around the tibia. In such situations, a split or bifurcated fixation portion 92 may be employed such as shown with implant 80D in the exemplary embodiment of FIG. 11D. In this embodiment, split fixation portion 92 forms two fixation arms 92A and 92B at its caudal end, one extending laterally and posteriorly, and a second extending medially and anteriorly, that can be configured to wrap around the tibia in opposing directions to the extent appropriate for the patient anatomy and clinical situation presented. These fixation arms 92A, 92B can be oriented transverse to longitudinal axis of fixation portion 92 to extend primarily in the medial-lateral direction, or angled caudally as wells as medially or laterally. Alternatively, a single arm 92A or 92B may be employed in a manner similar to the exemplary embodiments of FIGS. 11A and 11B employing single extended fixation portions 90A or 90B. In addition, the caudal ends of fixation arms 92A, 92B may have holes configured to receive a rod or screw extending through one fixation arm 92A and through the tibia to the other fixation arm 92B.

Again, depending on patient anatomy, it also may be desirable to provide differently shaped bone facing fixation surfaces for fixation portion 82. Alternatives include fixed protrusions 94 and adjustable protrusions 96 as illustrated in FIG. 11E. Such protrusions allow the fixation portion 82 to contact the bone at discreet locations to accommodate variability in the contour of the bone surface. Fixed protrusions may be ground to a desired height and shape according to the anatomy of each patient to provide further patient specific adaptability. Adjustable protrusions may be provided with an adjustment mechanism 98, such as a threaded member accessible at the outer surface of fixation portion 82 and rotatable to move adjustable protrusions 96 between an inner position and an outer position (shown in dashed lines in FIG. 11E) to adjust the distance the protrusion extends from the fixation portion 32. Alternatively or additionally, fixed or adjustable protrusions may be provided on the bone-facing side of the displacement portion such that it contacts the bone at discrete locations, accommodating variations in shape of the bone surface on the cranial side of the tibial tuberosity where the displacement portion extends under the patellar tendon.

The present disclosure contains multiple alternative embodiments and multiple alternative features within each disclosed embodiments. As will be apparent to persons of ordinary skill in the art based on the teachings herein contained, different features may be employed with embodiments other than those on which they are shown in the drawings for purposes of illustration. Given the number of possible combinations, it is not possible within a concise disclosure to separately illustrate each combination of features as would be understood by those skilled in the art. As non-limiting examples, each of the different supplemental fixation or support means shown in FIGS. 9A or 9C, the fixation base member shown in FIGS. 10A-E, and/or the different fixation portion shapes shown in FIGS. 11A-11E may be used together in different combinations or individually with each different implant herein.

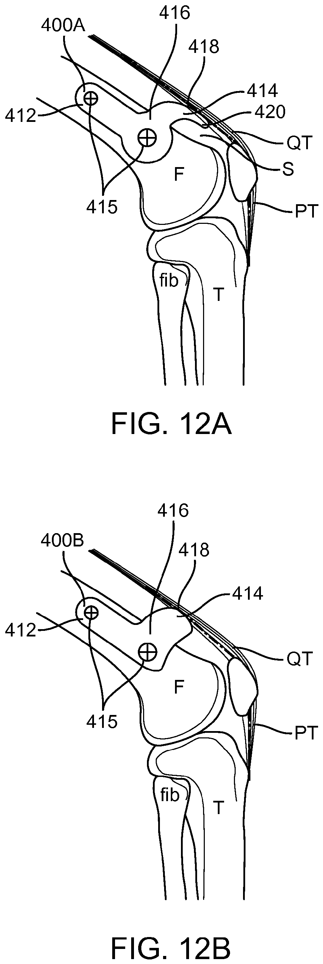

FIGS. 12A and 12B depict further exemplary embodiments of implants 400A and 400B, respectively, also for treating patellofemoral osteoarthritis and/or patellar maltracking, but with femorally mounted implants as shown. As with other embodiments disclosed herein, implants 400A and 400B each have a fixation portion 412 including one or more holes 415 for receiving screws, or other fixation means for anchoring the implant to bone. Fixation portion 412 is generally straight and elongated, being configured for positioning in general alignment with the femoral shaft on the lateral, medial or anterior-medial/lateral side of the femur, cranially with respect to the patella. Holes 415 may be positioned in approximate alignment with a longitudinal centerline of fixation portion 412 as shown, however additional holes for added fixation security, similar to embodiments shown, *inter alia,* FIGS. 8 or 11A-E. Preferably fixation portion 412 is configured to be mounted to the femur outside the joint capsule, cranially with respect to the tendons, ligaments and other tissues that form the capsule.

Displacement portion 414, is configured and dimensioned to be positioned under the quadriceps tendon caudally separated from the insertion point of the tendon in the quadriceps muscle and cranially with respect to its attachment point to the patella, with the entire displacement portion 414 preferably being disposed entirely outside the joint capsule. Thus, for a medially placed implant displacement portion will also extend laterally across an anterior portion of the femur. Likewise, a laterally placed device will have a displacement portion 414 that also extends medially across an anterior portion of the femur. The displacement portion 414 is configured to atraumatically engage the quadriceps tendon and displace it anteriorly relative to the femur, thus increasing space in the patellofemoral area. The displacement portion 414 has a width in the lateral-medial direction selected to accommodate the full width of the quadriceps tendon so that the tendon remains engaged along its entire width as it slides on the displacement portion. Displacement portion 414 has a length in the caudal-cranial direction selected so that it does not interfere with the patella. Displacement portion 414 preferably has a convex curvature on bearing surface 418, which engages the tendon. In general, the displacement portion and, in particular the bearing surface of the displacement portion, will be free of holes or other fixation means, with configuration similar to the bearing surfaces in other described embodiments. As with tibial mounted embodiments, the displacement portion 414 and/or bearing surface may have a curvature which provides a constant displacement of the tissue throughout the range of motion of the joint, or configured to vary the displacement at different points throughout the range of motion. The curvature may be entirely or partially spherical, elliptical, parabolic, logarithmic spiral, or other curvature or combination thereof. In preferred embodiments the displacement portion 414 is configured such that a cranial aspect of bearing surface 418 slopes or curves gradually away from the femur as it extends in the caudal direction to provide gradually increasing displacement of the tendon.

A spanning section 416 interconnects fixation portion 412 and displacement portion 414. Spanning section 416, is previously described to appropriately position the displacement portion with respect to the fixation portion and soft and bony tissues in the area of treatment. As with other embodiments, displacement of the target tissue can be altered by changing the length, curvature and angle of the spanning section among other features. Implant 400A (FIG. 12A) may also include a cantilevered portion 420 of displacement portion 414 which has an undercut surface that is spaced apart from the underlying femoral surface. As with other described embodiments, cantilevered portion 420 creates a space (S) between the implant and the bone to accommodate soft tissue structures as needed. Cantilevered portion 420 also extends the displacement portion 414 in the caudal direction toward the patella to optimize displacement while minimizing interference with the joint capsule and other soft tissues. Alternatively, implant 400B, shown in FIG. 12B, may be configured without a cantilevered portion such that substantially all of the bearing surface 418 overlies and is supported by portions of the displacement portion 414 that engage the femur.

Implants 400A, 400B may optionally include various other features described above in connection with tibial-mounted embodiments. For example, implants 400A and 400B may include a supporting section extending from a cranial part of the underside of the displacement portion into and merging with the fixation portion as described above in connection with tibially mounted embodiments. The configuration of a supporting section in these embodiments will, however, be generally inverted to accommodate fixation on the femur cranially with respect to the patella, as opposed to, on the tibia, caudally with respect to the patella. Implants 400A, 400B may alternatively have a tab or extension portion extending cranially, medially, and/or posteriorly from the displacement portion 414 on the opposite side from the fixation portion 412 which can engage the femur to provide additional support for the displacement portion 414. As with the embodiments shown in FIGS. 9A and 9C, such tab or extension may include a hole through which a bone screw may be inserted into the bone, or a hole for receiving a rod or screw extending from fixation portion 412 through the femur.

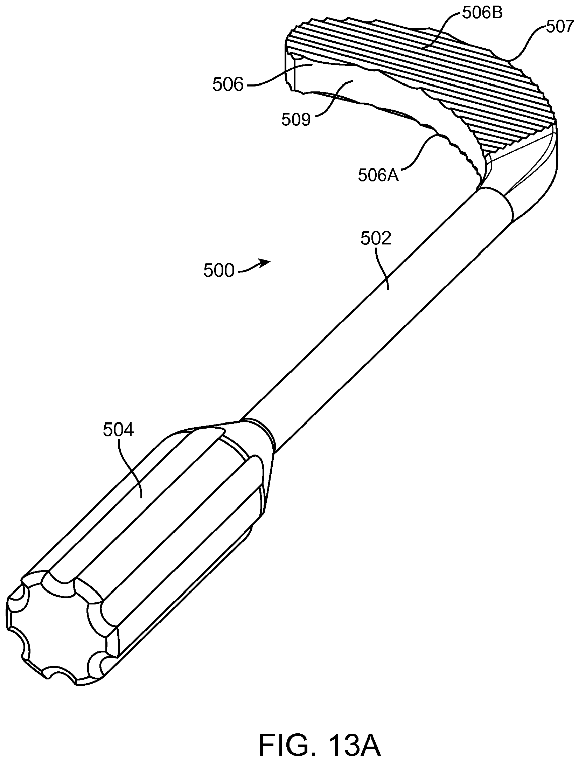

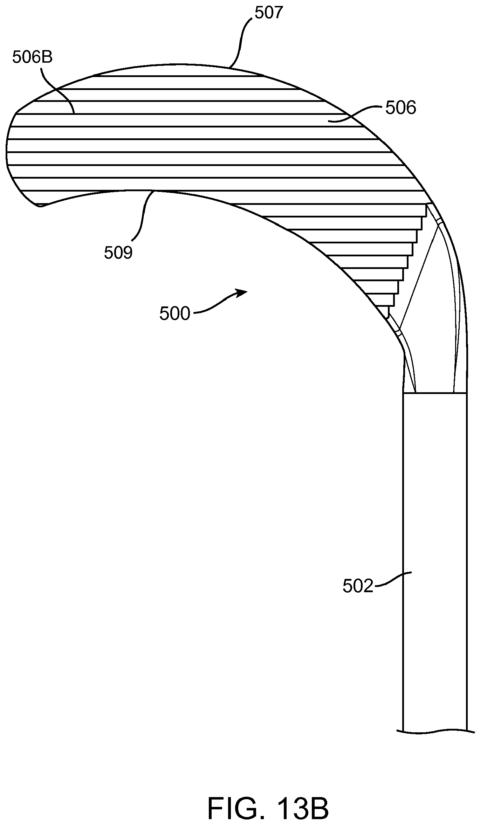

In general, implants according to embodiments of the present invention may be positioned and fixed using well-known instrumentation that is used for other orthopedic implant procedures. However, because of the unique position and seating of embodiments of the present invention, a specially shaped curved file as shown in FIGS. 13A and 13B may be useful for preparing the bone surface at the implant location, particularly for tibially mounted implants. Curved file 500 comprises a shaft 502 with a handle 504 at a proximal end and a curved file element 506 at the opposite, distal end. File element 506, preferably made of a metal suitable for filing bone, extends in a transverse direction from shaft 502 and has a lower surface 506A and an upper surface 506B, one or both of which have grooves, knurling, points, bumps, or other features configured to file the bone surface to give it a suitable shape for receiving the implants of the invention. File element 506 is preferably curved about a second axis extending in a direction transverse to shaft 502 longitudinal axis in an anterior-posterior direction, giving file element 506 a convex cranial side 507 and a concave caudal side 509. Upper and lower surfaces 506A, 506B are disposed at least partially in respective planes which are intersected by the second axis. In addition, lower and/or upper surfaces 506A, 506B may have a curvature about a third axis parallel to the longitudinal axis of shaft 502. This latter curvature may have various shapes, e.g. generally matching the curvature of the anterior surface of the tibia in the region just cranial to the tuberosity where the implant will be fixed, or a different curvature as is suitable to provide a stable base for the implant. Further, either or both the convex cranial side 507 or concave caudal side 509 may have grooves or other features to facilitate filing the bone with these surfaces. Moreover, the lower and/or upper surfaces 506A, 506B may have either a convex or concave curvature about an axis transverse to shaft 502 as may be suitable to create the particular shape desired for the bone surface. The file element 506 may be inserted through an incision on the lateral or medial side of the tibia just cranial to the tibial tuberosity such that the file element extends across the anterior tibial surface. File 500 may be drawn back and forth in a cranial-caudal direction (parallel to the tibial shaft), or in a medial-lateral direction, to file down the tibial surface cranially of the tuberosity to the desired shape. It should be noted that in addition to their use in implanting the implants of the invention, the files disclosed herein may be used for other treatments, including bone re-shaping for the treatment of Osgood-Schlatter disease or other diseases.

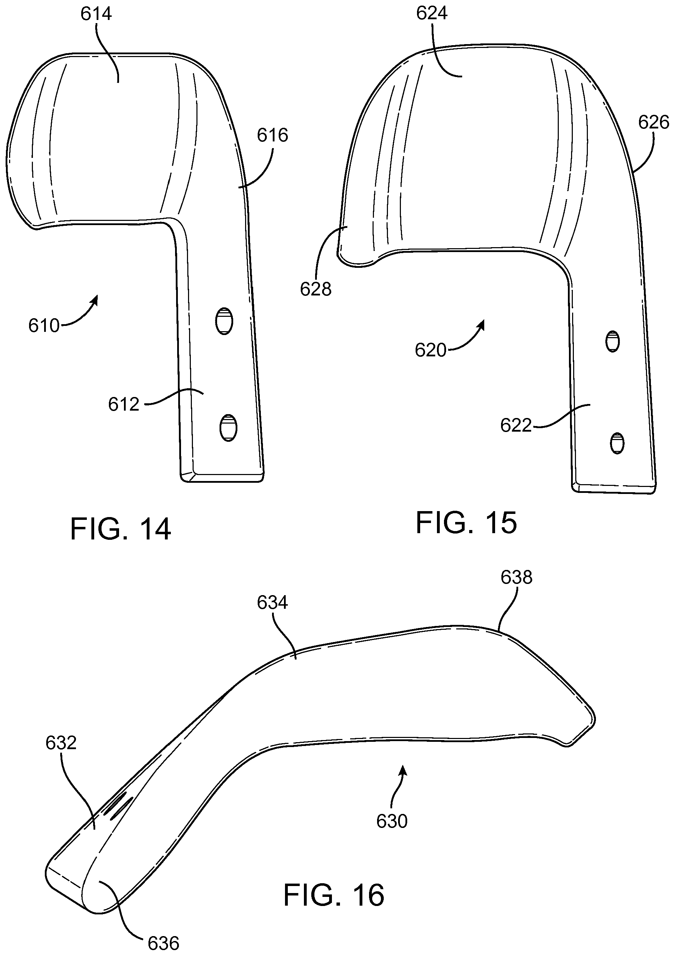

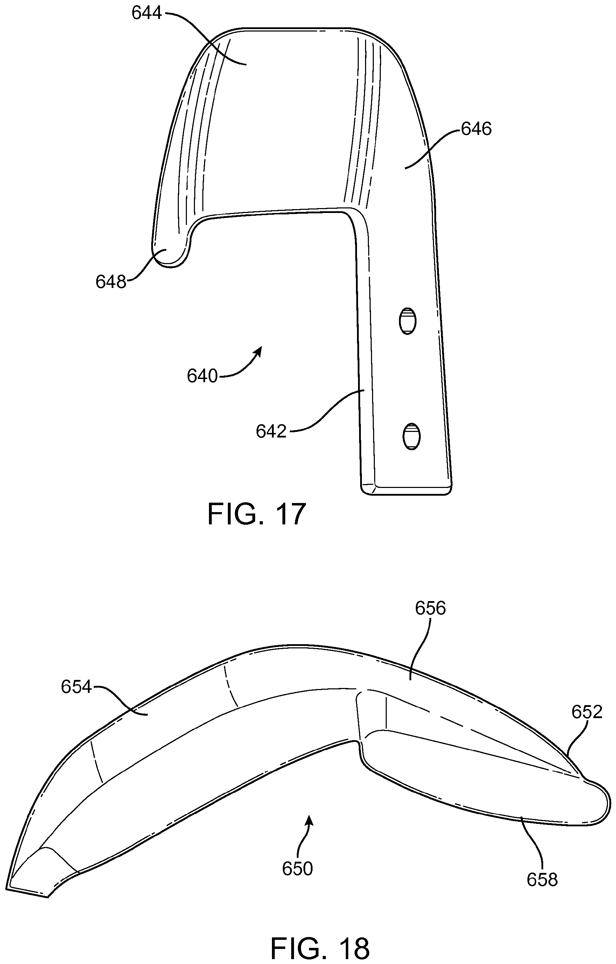

FIGS. 14-18 illustrate further exemplary implants made according to the teachings of the present disclosure. For example, implant 610 in FIG. 14 is made according to the general teachings of the disclosure and includes, *inter alia,* fixation portion 612, displacement portion 614 and spanning section 616, as well as other features described herein for the various embodiments. Implant 620, shown in FIG. 15, includes a fixation portion 622, displacement portion 624 and spanning section 626, wherein displacement portion 624 is extended laterally as compared to implant 610 so that the lateral edge 628 of displacement portion 624 may be supported on the tibia, for example on Gerdy's tubercle or adjacent thereto, to provide a supplemental support element. In this regard, implant 620 includes features similar to embodiments shown in FIGS. 7A-D. Implant 630, shown in FIG. 16, also includes a fixation portion 632, displacement portion 634 and spanning section 636. Displacement portion 634 has a gradually increased thickness area 638 towards the lateral end in order to increase the tendon displacement at the lateral side. Implant 640, shown in FIG. 17, includes fixation portion 642, displacement portion 644 and spanning section 646. In this embodiment, displacement portion 644 includes lateral pad 648 at an outer end to provide supplemental support and fixation adjacent the tibial tubercle. In this regard, implant 640 may include features similar to embodiments shown in FIGS. 9C and 9D. Additionally, lateral pad 648 may be designed to hook the tibial tubercle. Implant 650, shown in FIG. 18, includes fixation portion 652, displacement portion 654 and spanning section 656. Fixation portion 652 has a bone engaging surface 658 formed with a convex profile to facilitate positioning along the tibial tuberosity prior to fixation. The convex profile may be in the range of about 10 degrees of convexity.

Exemplary embodiments have been disclosed above and illustrated in the accompanying drawings. It will be understood by those skilled in the art that various changes, omissions and additions may be made to that which is specifically disclosed herein without departing from the scope of the present invention as defined by the appended claims.

## Claims

1. A prosthesis for repositioning a target tissue, the target tissue comprising a connective tissue or muscle relative to a bone on which said target tissue acts, and the prosthesis comprising:
a fixation portion (38) having one or more fixation features (39) configured to receive fixation elements for securing the implant to the bone; and
a displacement portion (32) having a first end connected to the fixation portion and a free end (33) opposite the first end, the displacement portion having a bearing surface (34) configured to atraumatically engage and reposition the target tissue relative to the bone;
wherein the displacement portion has a base portion configured to engage the bone, **characterised in that** the displacement portion also has a cantilevered portion (36) extending from the base portion to the free end, the cantilevered portion being configured to be spaced apart from bone when the base portion is engaging the bone.

2. The prosthesis of claim 1, wherein the displacement portion is at least partially ramp-shaped such that, when implanted, a lower part of the displacement portion has a first height relative to the bone surface and an upper part of the displacement portion has a second greater height relative to the bone surface, the ramp-shaped portion facing in a direction generally away from the free end.

3. The prosthesis of claim 1, wherein the displacement portion is connected to the fixation portion at an end opposite the free end by a spanning section.

4. The prosthesis of claim 3 wherein:
the fixation portion is configured to be mounted to the tibia proximate the upper tibial extremity and medially or laterally of the tibial tuberosity;
the spanning section is configured and dimensioned to extend cranially and laterally or medially from the fixation portion in a direction towards the tibial mid-line;
the displacement portion is configured and dimensioned to extend from the spanning section further laterally or medially under patellar tendon and in engagement therewith, and to displace the patellar tendon at least anteriorly from a normal, anatomical path; and
a supplemental support element with a bone engaging surface disposed at an end of the displacement portion opposite the spanning section, the displacement portion being further configured and dimensioned to dispose said bone engaging surface against the tibial surface when the fixation portion is mounted to the tibia;
the displacement portion being supported and positioned by the supplemental support element, the spanning section and fixation portion such that said displacement is sufficient to alter the location, angle or magnitude of forces exerted thereby on the patella so as to achieve a therapeutic effect in patellofemoral compartment of the knee.

5. The prosthesis of claim 4, wherein the therapeutic effect comprises a reduction of loading on an articular surface in the patellofemoral compartment of the knee.

6. The prosthesis of claims 3, 4 or 5, wherein said displacement portion and spanning section are configured and dimensioned in combination to displace the patellar tendon from a pre-treatment anatomical path by displacement distance of more than about 5 mm and less than about 30 mm when the fixation portion is mounted to the tibia.

7. The prosthesis of claims 3-6, wherein said displacement portion and spanning section are further configured to position the displacement portion to define a space between at least a part of the displacement portion and the tibial surface.

8. The prosthesis of claim 7, wherein said space is further defined by a cranially-facing undercut in the displacement portion.

9. The prosthesis of claims 4-8, further comprising a bone fixation element cooperating with said supplemental support element.

10. The prosthesis of claim 9, wherein said bone fixation element comprises at least one bone screw hole in said supplemental support element.

11. The prosthesis of claims 4-10, wherein said displacement portion and supplemental support element lie along a displacement portion axis at an angle of about 80-135 degrees with respect to a fixation portion axis.

12. The prosthesis of claims 1-10, wherein the displacement portion extends further in a caudal direction, with the displacement portion configured to extend around the tibia to an opposite side of the tibia from the fixation portion.

13. The prosthesis of any preceding claim, wherein:
the fixation portion comprises a body member and a separate base member to which the body member may be coupled, said body and base members having complementary, mating shapes to provide a secure coupling of the body member to the base member; and
the separate base member includes plural bone screw holes for fixation to the tibia.

14. The prosthesis of claim 13, wherein the fixation portion body member includes at least one bone screw access hole positioned to align with a bone screw hole in the fixation portion base member when said body member is fit with the base member.

15. The prosthesis of any of claims 3-14, wherein:
the fixation portion is disposed at an angle with the spanning section such that with the prosthesis implanted and the displacement portion engaging the patellar tendon, the fixation portion is substantially aligned with the tibial shaft;
the spanning section is configured and dimensioned to avoid contact with the medial edge of the patellar tendon; and
the displacement portion is further configured and dimensioned to lie substantially parallel to the axis of the tibial plateau when the fixation portion is secured medially of the tibial tuberosity with the fixation portion substantially aligned with the tibial shaft.

## Patentansprüche

1. Prothese zur Reponierung eines Zielgewebes in Relation zu einem Knochen, auf welchen dieses Zielgewebe wirkt, wobei das Zielgewebe ein Bindegewebe oder einen Muskel umfasst, und wobei die Prothese umfasst:
einen Fixierungsabschnitt (38), der einen oder mehrere Fixierungsmerkmale (39) umfasst, die so eingerichtet sind, dass sie Fixierungselemente zur Sicherung des Implantats an dem Knochen umfassen; und
einen Verschiebungsabschnitt (32), welcher ein erstes Ende, das mit dem Fixierungsabschnitt verbunden ist, sowie ein freies Ende (33) aufweist, das sich gegenüber dem ersten Ende befindet, wobei der Verschiebungsabschnitt eine Lagerfläche (34) aufweist, welche so eingerichtet ist, dass sie auf atraumatische Weise an dem Zielgewebe angreift und es in Relation zu dem Knochen reponiert;
wobei der Verschiebungsabschnitt einen Basisabschnitt aufweist, welcher so eingerichtet ist, dass er an dem Knochen angreift, **dadurch gekennzeichnet, dass** der Verschiebungsabschnitt auch einen auskragenden Abschnitt (36) aufweist, der sich von dem Basisabschnitt zu dem freien Ende hin erstreckt, wobei der auskragende Abschnitt so eingerichtet ist, dass er in einem Abstand zu dem Knochen angeordnet ist, wenn der Basisabschnitt an dem Knochen angreift.

2. Prothese gemäß Anspruch 1, wobei der Verschiebungsabschnitt mindestens teilweise rampenförmig ist, so dass bei Implantierung ein unterer Teil des Verschiebungsabschnitts eine erste Höhe in Relation zu der Knochenoberfläche aufweist und ein oberer Teil des Verschiebungsabschnitts eine zweite, größere Höhe in Relation zu der Knochenoberfläche aufweist, wobei der rampenförmige Abschnitt in eine Richtung ausgerichtet ist, die im Allgemeinen von dem freien Ende weg weist.

3. Prothese gemäß Anspruch 1, wobei der Verschiebungsabschnitt durch einen Spannabschnitt mit dem Fixierungsabschnitt an einem gegenüber dem freien Ende befindlichen Ende verbunden ist.

4. Prothese gemäß Anspruch 3, wobei:
der Fixierungsabschnitt so eingerichtet ist, dass er an dem Schienbein proximal zur oberen Tibiaextremität und medial oder lateral zur Tuberositas tibiae gelagert wird;
der Spannabschnitt so eingerichtet und dimensioniert ist, dass er sich kranial oder lateral oder medial von dem Fixierungsabschnitt in eine Richtung zu der Mittellinie des Schienbeins hin erstreckt;
der Verschiebungsabschnitt so eingerichtet und dimensioniert ist, dass er sich von dem Spannabschnitt weiter lateral oder medial unter der Patellasehne und an diese angreifend erstreckt, und zwar derart, dass er die Patellasehne von einem normalen, anatomischen Pfad mindestens in die anteriore Richtung verschiebt; und
ein ergänzendes Stützelement mit einer an Knochen engreifenden Oberfläche, das an einem Ende des Verschiebungsabschnitts gegenüber dem Spannabschnitt angeordnet ist, wobei der Verschiebungsabschnitt ferner so eingerichtet und dimensioniert ist, dass er die an Knochen engreifende Oberfläche an der Schienbeinoberfläche anordnet, wenn der Fixierabschnitt an dem Schienbein gelagert ist;
wobei der Verschiebungsabschnitt durch das ergänzende Stützelement, den Spannabschnitt und den Fixierabschnitt so gestützt und positioniert wird, dass die Verschiebung ausreicht, um die Position, den Winkel oder die Größe der Kräfte zu verändern, die dadurch auf die Patella ausgeübt werden, so dass ein therapeutischer Effekt im patellofemoralen Kompartiment des Knies erzeugt wird.

5. Prothese gemäß Anspruch 4, wobei der therapeutische Effekt eine Reduktion der Belastung auf eine Gelenkoberfläche n dem patellofemoralen Kompartiment des Knies umfasst.

6. Prothese gemäß den Ansprüchen 3, 4 oder 5, wobei der Verschiebungsabschnitt und der Spannabschnitt so eingerichtet und dimensioniert sind, dass sie in Kombination die Patellasehne aus einem vor der Behandlung vorliegenden anatomischen Pfad um eine Verschiebungsstrecke von mehr als ungefähr 5 mm und weniger als ungefähr 30 mm verschieben, wenn der Fixierabschnitt an dem Schienbein gelagert ist.

7. Prothese gemäß den Ansprüchen 3 bis 6, wobei der Verschiebungsabschnitt und der Spannabschnitt ferner so eingerichtet sind, dass sie den Verschiebungsabschnitt so positionieren, dass ein Raum zwischen mindestens einem Teil des Verschiebungsabschnitts und der Schienbeinoberfläche definiert wird.

8. Prothese gemäß Anspruch 7, wobei der Raum ferner durch einen kranial ausgerichteten Hinterschnitt in dem Verschiebungsabschnitt definiert wird.

9. Prothese gemäß Ansprüchen 4 bis 8, ferner ein Knochenfixierungselement umfassend, das mit dem ergänzenden Stützelement zusammenwirkt.

10. Prothese gemäß Anspruch 9, wobei das Knochenfixierungselement mindestens ein Knochenschraubenloch in dem ergänzenden Stützelement umfasst.

11. Prothese gemäß Ansprüchen 4 bis 10, wobei der Verschiebungsabschnitt und das ergänzende Stützelement entlang einer Verschiebungsabschnittachse mit einem Winkel von ungefähr 80 bis 135 Grad in Bezug auf die Fixierabschnittachse angeordnet sind.

12. Prothese gemäß Ansprüchen 1 bis 10, wobei sich der Verschiebungsabschnitt ferner in eine kaudale Richtung erstreckt, wobei der Verschiebungsabschnitt so eingerichtet ist, dass er sich von dem Fixierabschnitt um das Schienbein herum zu einer entgegengesetzten Seite des Schienbeins erstreckt.

13. Prothese gemäß einem beliebigen der vorhergehenden Ansprüche, wobei:
der Fixierabschnitt ein Körperbauteil und ein separates Basisbauteil umfasst, an welches das Körperbauteil angekoppelt werden kann, wobei Körperbauteil und Basisbauteil sich ergänzende, zusammenpassende Formen aufweisen, um eine sichere Kopplung des Körperbauteils an das Basisbauteil sicherzustellen; und
das separate Basisbauteil mehrere Knochenschraubenlöcher zur Fixierung an dem Schienbein umfasst.

14. Prothese gemäß Anspruch 13, wobei das Fixierabschnitt-Körperbauteil mindestens ein Knochenschrauben-Zugriffsloch umfasst, das so positioniert ist, dass es an einem Knochenschraubenloch in dem Fixierabschnitt-Körperbauteil ausgerichtet ist, wenn das Körperbauteil mit dem Basisbauteil zusammengefügt ist.

15. Prothese gemäß einem beliebigen der Ansprüche 3 bis 14, wobei:
der Fixierabschnitt in einem Winkel mit dem Spannabschnitt angeordnet ist, so dass bei implantierter Prothese und bei an der Patellasehne angreifendem Verschiebungsabschnitt der Fixierabschnitt im Wesentlichen am Tibiaschaft ausgerichtet ist;
der Spannabschnitt so eingerichtet und dimensioniert ist, dass er Kontakt mit der medialen Kante der Patellasehne vermeidet; und
der Verschiebungsabschnitt ferner so eingerichtet und dimensioniert ist, dass er im Wesentlichen parallel zu der Achse des Tibiaplateaus liegt, wenn der Fixierabschnitt medial zur Tuberositas tibiae gesichert ist, wobei der Fixierabschnitt im Wesentlichen an dem Tibiaschaft ausgerichtet ist.

## Revendications

1. Prothèse de repositionnement d'un tissu cible, le tissu cible comprenant un tissu conjonctif ou un muscle associé à un os sur lequel ledit tissu cible agit, et la prothèse comprenant :
une partie de fixation (38) présentant un ou plusieurs éléments de fixation (39) configurés pour recevoir des organes de fixation afin de fixer l'implant sur l'os ; et
une partie de déplacement (32) présentant un première extrémité reliée à la partie de fixation et une extrémité libre (33) opposée à la première extrémité, la partie de déplacement présentant une surface de soutien (34) configurée pour s'engager avec le tissu cible et le repositionner de manière atraumatique par rapport à l'os ;
où la partie de déplacement présente une partie de base configurée pour s'engager avec l'os,
**caractérisée en ce que** la partie de déplacement présente également une partie en porte-à-faux (36) s'étendant à partir de la partie de base jusqu'à l'extrémité libre, la partie en porte-à-faux étant configurée de sorte à être écartée de l'os lorsque la partie de base est engagée avec l'os.

2. Prothèse selon la revendication 1, dans laquelle la partie de déplacement est façonnée au moins partiellement selon une forme de voûte de telle sorte que, lorsqu'elle est implantée, une portion inférieure de la partie de déplacement présente une première hauteur par rapport à la surface de l'os et une portion supérieure de la partie de déplacement présente une deuxième hauteur plus grande par rapport à la surface de l'os, la partie façonnée en forme de voûte étant orientée selon une direction globalement opposée à l'extrémité libre.

3. Prothèse selon la revendication 1, dans laquelle la partie de déplacement est reliée à la partie de fixation au niveau d'une extrémité opposée à l'extrémité libre par une section de chevauchement.

4. Prothèse selon la revendication 3, dans laquelle :
la partie de fixation est configurée pour être montée sur le tibia à proximité de l'extrémité tibiale supérieure et de manière médiale ou latérale par rapport à la tubérosité tibiale ;
la section de chevauchement est configurée et dimensionnée pour s'étendre de manière craniale et latérale ou médiale à partir de la partie de fixation dans une direction vers la ligne médiane du tibia ;
la partie de déplacement est configurée et dimensionnée pour s'étendre à partir de la section de chevauchement de manière davantage latérale ou médiale sous un tendon rotulien et en prise avec celui-ci, et pour déplacer le tendon rotulien au moins vers le côté antérieur à partir d'une trajectoire anatomique normale ; et
un élément de support supplémentaire ayant une surface d'engagement d'os disposé au niveau d'une extrémité de la partie de déplacement opposée à la section de chevauchement, la partie de déplacement étant en outre configurée et dimensionnée pour disposer ladite surface d'engagement d'os contre la surface tibiale lorsque la partie de fixation est montée sur le tibia ;
la partie de déplacement étant supportée et positionnée par l'élément de support supplémentaire, la section de chevauchement et la partie de fixation de telle sorte que ledit déplacement est suffisant pour modifier l'emplacement, l'angle ou l'ampleur des forces exercées par celle-ci sur la rotule de manière à obtenir un effet thérapeutique dans le compartiment fémoro-patellaire du genou.

5. Prothèse selon la revendication 4, dans laquelle l'effet thérapeutique comprend une réduction de la charge sur une surface articulaire dans le compartiment fémoro-patellaire du genou.

6. Prothèse selon les revendications 3, 4 ou 5, dans laquelle lesdites partie de déplacement et section de chevauchement sont configurées et dimensionnées en combinaison pour déplacer le tendon rotulien d'une trajectoire anatomique de prétraitement d'une distance de déplacement supérieure à environ 5 mm et inférieure à environ 30 mm lorsque la partie de fixation est montée sur le tibia.

7. Prothèse selon les revendications 3 à 6, dans laquelle lesdites partie de déplacement et section de chevauchement sont en outre configurées pour positionner la partie de déplacement de manière à définir un espace entre au moins une portion de la partie de déplacement et la surface tibiale.

8. Prothèse selon la revendication 7, dans laquelle ledit espace est en outre défini par une découpe orientée de manière craniale dans la partie de déplacement.

9. Prothèse selon les revendications 4 à 8, comprenant en outre un élément de fixation d'os coopérant avec ledit élément de support supplémentaire.

10. Prothèse selon la revendication 9, dans laquelle ledit élément de fixation d'os comprend au moins un trou pour vis à os dans ledit élément de support supplémentaire.

11. Prothèse selon les revendications 4 à 10, dans laquelle lesdits partie de déplacement et élément de support supplémentaire se situent le long d'un axe de partie de déplacement à un angle d'environ 80 à 135 degrés par rapport à un axe de partie de fixation.

12. Prothèse selon les revendications 1 à 10, dans laquelle la partie de déplacement s'étend en outre dans une direction caudale, la partie de déplacement étant configurée de manière à s'étendre autour du tibia vers un côté opposé du tibia à partir de la partie de fixation.

13. Prothèse selon l'une quelconque des revendications précédentes, dans laquelle :
la partie de fixation comprend un élément de corps et un élément de base séparé auquel l'élément de corps peut être couplé, lesdits éléments de corps et de base ayant des formes d'accouplement complémentaires pour fournir un couplage sûr de l'élément de corps à l'élément de base ; et
l'élément de base séparé comprend plusieurs trous pour vis à os pour une fixation sur le tibia.

14. Prothèse selon la revendication 13, dans laquelle l'élément de corps de la partie de fixation comprend au moins un trou d'accès pour vis à os positionné de manière à être aligné avec un trou pour vis à os dans l'élément de base de la partie de fixation lorsque ledit élément de corps est installé sur l'élément de base.

15. Prothèse selon l'une quelconque des revendications 3 à 14, dans laquelle :
la partie de fixation est disposée à un certain angle par rapport à la section de chevauchement de telle sorte que, lorsque la prothèse est implantée et que la partie de déplacement s'engage avec le tendon rotulien, la partie de fixation est globalement alignée avec la diaphyse tibiale ;
la section de chevauchement est configurée et dimensionnée pour éviter tout contact avec le bord médial du tendon rotulien ; et
la partie de déplacement est en outre configurée et dimensionnée pour se situer globalement parallèlement à l'axe du plateau tibial lorsque la partie de fixation est fixée de manière médiale par rapport à la tubérosité tibiale, la partie de fixation étant globalement alignée avec la diaphyse tibiale.
